# EUROPÄISCHE PATENTSCHRIFT

(11) **EP 0 251 332 B1**
(45) Veröffentlichungstag und Bekanntmachung des Hinweises auf die Patenterteilung: **20.10.1993**
(21) Anmeldenummer: 87109616.0
(22) Anmeldetag: 03.07.1987
(51) Int. Cl.: A61F 13/15

(54) **Wegwerfwindel**
Disposable diaper
Couche à jeter

(30) Priorität: 04.07.1986 JP 158609/86; 20.08.1986 JP 194500/86
(43) Veröffentlichungstag der Anmeldung: 07.01.1988
(73) Patentinhaber: UNI-CHARM CORPORATION, Kawanoe-shi Ehime-ken (JP)
(72) Erfinder: Suzuki, Migaku, Kawanoe-shi Ehime-ken (JP); Ochi, Mitzuso, Uma-gun Ehime-ken (JP); Kudo, Takeshi, Ehime-ken (JP)
(74) Vertreter: Sperling, Rüdiger, Dipl.-Ing.

(56) Entgegenhaltungen:
- EP-A- 0 219 326
- FR-A- 2 388 515
- FR-A- 2 554 325
- FR-A- 2 572 902
- GB-A- 2 161 059
- US-A- 4 579 556

## Beschreibung

Die Erfindung betrifft eine Wegwerfwindel mit einem Saugkern vorbestimmter Dicke, einer zumindest die obere Fläche des Saugkerns bedeckende wasserdurchlässige obere Lage, einer zumindest die Boden- oder rückwärtige Fläche des Saugkerns bedeckende wasserundurchlässige rückseitige Lage, mit wasserundurchlässigen Seitenklappen, die sich an gegenüberliegenden Seiten des Saugkerns erstrecken, ersten elastischen Mitteln, die entlang der jeweiligen Seitenklappen elastische Einkräuselungen bilden und mit nach innen (zum Körper) ragenden umgekehrten U-oder Ω-förmig ausgebildeten Schutzwänden.

Aus der GB-A-2 161059 ist eine Wegwerfwindel mit Sperrwänden und einem Saugkern bekannt. Der Saugkern besitzt eine im wesentlichen halbmondförmige Querschnittsform, wobei die Enden der Monde sich unmittelbar im Bereich der Verbindungsnähte zwischen der oberen, wasserdurchlässigen und unteren wasserundurchlässigen Lage erstrecken. Die Sperrwände, sie können, was anschließend deutlich wird, nicht als Leckschutzwände bezeichnet werden, erstrecken sich nicht wie bei der Erfindung seitlich des Saugkerns, sondern **oberhalb** des Saugkerns und werden jeweils von einer Falte der oberen Lage gebildet. Da die obere Lage eine wasserdurchlässige Lage ist, können folglich diese Sperrwände auch nicht wasserundurchlässig sein. Sie halten lediglich feste Ausscheidungen zurück. Daher können sie nicht als Leckschutzwände bezeichnet werden. Über eine besondere Form der elastischen Bänder in den Scheiteln der Sperrwände sind aus der Schrift keine Angaben zu entnehmen.

Aus der EP-A-0 219 326, Entgegenhaltung 4, ist eine Wegwerfwindel bekannt, bei der der Saugkern die Gestalt einer Sanduhr aufweist; die im Abstand voneinander parallel angeordneten Sperrwände verlaufen somit auf der Bauchseite auf dem Saugkern, schneiden zu Beginn des Schrittbereichs die Umrandung des Saugkerns und Verlaufen dann im Schrittbereich in einem Abstand vom Saugkern, um anschließend im Gesäßbereich wieder auf dem Saugkern zu verlaufen. Sie schließen daher den Saugkern an den Seitenwänden nicht hermetisch ab. Auch sind die elastischen Mittel zum Aufrichten der Sperrwände flach in der Krone der Wand eingeklebt und weisen keine omegaförmige Gestalt auf.

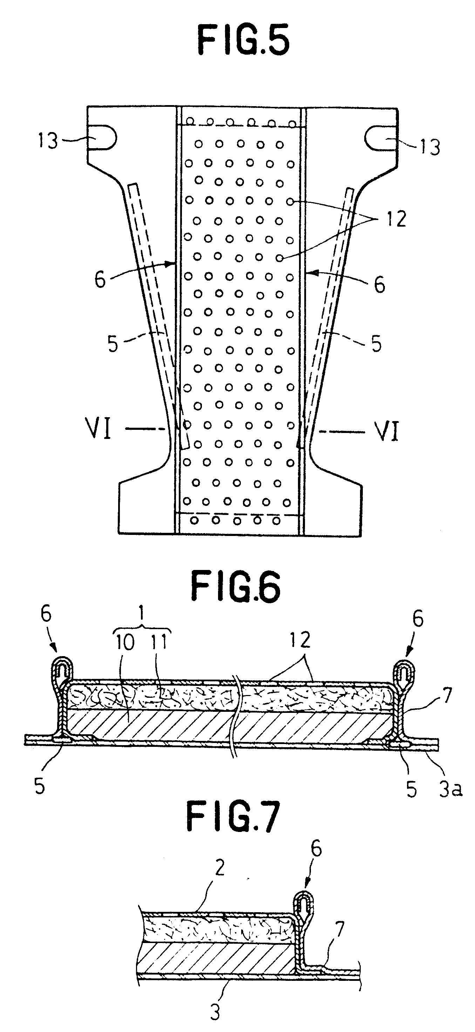

Bei dem in Figur 6 dargestellten Ausführungsbeispiel dieser Entgegenhaltung ist der Saugkern nach oben geknickt und am Außenbereich erst eine gefaltete Begrenzung aus dem Material der Außenlage angeordnet, wobei der hochgefaltete Saugkernabschnitt gemeinsam mit der gefalteten Begrenzung von einer durchlässigen Lage überdeckt.

Diese Entgegenhaltung ist lediglich im Rahmen des Artikels 54(3 und 4) zu berücksichtigen.

In der FR-A-2 388 515 ist eine Wegwerfwindel ohne die Schenkel eines Trägers umgebende Seitenklappen bekannt, wobei die sich gegenüberliegenden Seiten einer undurchlässigen Rückseite, die sich auf der Unterseite eines Saugkerns erstrecken, nach oben gefaltet sind, um diese sich gegenüberliegenden Seiten des Saugkerns zu bedecken. Die Ränder der einander gegenüberliegenden Seiten einer durchlässigen oberen Lage sind in Längsrichtung angeordnet, flach ausgeführt und mit einem elastischen Band versehen, um Einkräuselungen zu schaffen. Die Lehre dieser Patentanmeldung basiert im wesentlichen darauf, daß die jeweiligen Zonen der oberen Lage und der rückseitigen Lage, in denen die elastischen Bänder an beiden Lagen anhaften, von dem Saugkern freigesetzt sind und dadurch die Elastizität der elastischen Bänder frei von jeglicher einschränkenden Einwirkung durch die Steifigkeit des Kerns bleiben.

In der US-A-4 579 556 ist ein absorbierender Artikel, wie z.B. eine Wegwerfwindel mit Seitenklappen offenbart, die die Schenkel des Trägers eng umschließen, wobei die Seitenklappen durch Falten gegenüberliegender Seiten einer undurchlässigen rückwärtigen Lage gebildet werden, welche Rücklage an der Unterseite eines Saugkerns so angeordnet ist, daß sie sich an den einander gegenüberliegenden Seitenrändern des Saugkerns nach außen erstreckt. Die gefalteten Seitenränder sind an einer durchlässigen oberen Lage befestigt, die auf der Oberseite des Saugkerns angeordnet ist. Die Seitenklappen sind mit einem in Längsrichtung elastischen Band versehen, so daß sie durch die Elastizität dieser Bänder entlang Bereichen neben dem Saugkern aufrecht stehen. Die Erfindung ist allgemein darin zu sehen, daß Körperflüssigkeiten vor einem Austreten entlang der einander gegenüberliegenden Seitenränder des Saugkerns bewahrt werden und daß der Sitz des Gegenstands am Körper eines Trägers verbessert wird.

Im folgenden werden allgemeine Probleme diskutiert, die bei einer Windel auftreten.

In Wegwerfwindeln wird allgemein flockige Pulpe als Hauptmaterial für den Saugkern verwendet, wobei verschiedene Maßnahmen entwickelt worden sind, um die Saugfähigkeit zu verbessern, beispielsweise Bossieren und Vermischen mit hochabsorbierenden Polymerteilchen. Die Saugfähigkeitsrate von oberen Lagen und des Saugkerns sind nicht adequat hoch, um eine Urinmenge gleichzeitig aufzunehmen, sobald die Flüssigkeitsmenge auf die Oberfläche der oberen Lage und den Saugkern ausgeschieden wird, so daß die Flüssigkeit, zumindest teilweise, auf der Oberfläche dieser Lage zur Seite fließt und oft entlang den gegenüberliegenden Rändern der Windel austritt. Es ist offensichtlich, daß ein derartiges Ausfließen besonders leicht dann auftreten kann, wenn die ausgeschiedene Menge besonders groß ist.

In der FR-PS 2 388 515 ist lediglich vorgesehen, daß die elastischen Bänder in engem aber flachem Kontakt mit den Schenkein des Trägers kommen, um ein Austreten der Exkretion zu verhindern. Demzufolge bilden sich zwischen den Zonen der Windel, in denen die Bänder eingearbeitet sind und der Haut des Trägers Spalten, wenn die Tragebedingung der Windel von der korrektion Position abweicht, selbst dann, wenn diese Abweichung gering ist oder der Träger bestimmte Lagen einnimmt, wenn er sich bewegt. Demzufolge besteht die Möglichkeit, daß nicht nur eine beträchtliche Menge Urin seitlich austritt, sondern auch flüssige Exkremente durch diese Schlitze oder Spalten hindurchtreten.

Im Fall der Erfindung, die in der US-A-4 579 556 offenbart ist, sind die sich gegenüberliegenden Seitenränder des Saugkerns mit einem Teil der undurchlässigen rückwärtigen Lage bedeckt und man erhält dadurch einen gewissen Grad eines Leckageschutzes. Aber, wie bereits oben zum Ausdruck gebracht, verbleibt dennoch die Möglichkeit, daß, wenn die Urinmenge, die auf einmal auf die Oberfläche der Windel, unter der unmittelbar der Saugkern angeordnet ist, abgegeben wird, eine Teilmenge des Urins seitlich auf der oberen Lage abfließt und entlang den gegenüberliegenden Rändern der Windel austritt, bevor diese Menge wirksam durch den Saugkern aufgesaugt und mittels der undurchlässigen Seitenränder am Weiterfließen gehindert wird. Obwohl die Seitenklappen von der Elastizität der elastischen Bänder an den sich gegenüberliegenden Rändern des Saugkerns aufrechtgehalten werden, werden diese Seitenklappen zwangsläufig bezüglich der Seitenränder des Saugkerns nach außen geklappt, wenn die Windel angelegt ist, weil diese Seitenklappen dem Körperdruck ausgesetzt sind, so daß es schwierig ist, diesen Leckageschutz tatsächlich zu erwarten.

Der Erfindung liegt die Aufgabe zugrunde, eine Wegwerfwindel der genannten Art zu schaffen, die bei einfachem und kostengünstigem Aufbau sicher eine Leckage von Ausscheidungen, auch bei lebhafter Bewegung des Trägers der Windel zu verhindern.

Die Aufgabe wird erfindungesgemäß durch die im Hauptanspruch angegebene Lehre gelöst, wobei in den Unteransprüchen weiter vorteilhafte Ausgestaltungen angegeben sind.

Es wird somit erfindungsgemäß eine Wegwerfwindel bereitgestellt mit einem Saugkern einer vorbestimmten Dicke, einer zumindest die obere Fläche des Saugkerns bedeckenden oberen Lage, einer zumindest die Bodenfläche des Saugkerns bedeckenden rückseitigen Lage, wasserundurchlässigen Seitenklappen, die sich an gegenüberliegenden Seiten des Saugkerns nach außen erstrecken, ersten elastischen Bändern, die elastische Einkräuselungen entlang der jeweiligen Seitenklappen und Leckschutzwänden, die entlang der sich gegenüberliegenden Rändern erstrecken, auszubilden, wobei die Leckschutzwände von den oberen Rändern der sich gegenüberliegenden Seiten des Saugkerns durch zugeordnete zweite in Längsrichtung elastische Bänder aufrechtgehalten werden.

Erfindungsgemäß tritt keine Leckage entlang der sich gegenüberliegenden Seiten des Saugkerns auf, selbst wenn flüssige Exkretion von dem Saugkern aufgesaugt worden ist und dann die gegenüberliegenden Seitenränder des Saugkerns erreicht, da diese Seiten des Saugkerns mit einer wasserdichten Lage bedeckt sind. Selbst in einer Situation, in der flüssige Exkretion über die Oberfläche des Saugkerns fließt und seitlich zu den Seitenkanten abfließt, bevor es von dem Kern aufgesaugt werden kann, kommt es nicht zu einer Leckage, da die sich gegenüberliegenden Seiten des Saugkerns mit einer wasswerundurchlässigen Leckageschutzwand versehen sind, die sich jeweils an den oberen Kanten der jeweiligen Seiten erstrecken. Diese Seitenschutzwände bilden jeweils Einkräuselungen und schützen daher vor einer Leckage in diesen Bereichen. Sollte tatsächliche flüssige Exkretion über diese Leckageschutzwände zu den jeweiligen Seitenklappen, die außerhalb dieser Leckageschutzwände liegen - dies kann vielleicht durch eine heftige Bewegung oder eine andere außergewöhnliche Situation geschehen - so ist diese Leckage geringer als in einem Fall, in welchem diese Leckageschutzwände nicht vorhanden wären; diese Leckage kann durch die Seitenklappen, welche die Schenkel des Trägers wegen der Elastizität des in diesen Seitenklappen eingearbeiteten elastischen Bandes eng umschließen, an einem Weiterfließen gehindert werden.

Obwohl die Leckschutzwände dazu neigen, nach außen zu kippen oder unter dem Körperdruck des Trägers zusammenzufallen, richten die elastischen Bänder, die in den jeweiligen Seitenklappen eingearbeitet sind, diese Leckschutzwände auf und halten sie aufrecht, so daß die Möglichkeit, daß diese Leckschutzwände außer Kontakt mit der Haut des Trägers kommen und sich Spalten zwischen der Haut und den Leckschutzwänden bilden können, reduziert ist.

Die zweiten elastischen Bänder, die Teil der Leckschutzwände sind, werden in einem bestimmten Ausmaß in gedehntem Zustand gehalten, und zwar solange die Windel angelegt ist, wobei der Träger frei von jeglichem Unwohlgefühl ist und Falten der oberen Lage und des Kerns verhindert werden, die eine Leckage herbeiführen könnten.

Im folgenden wird die Erfindung anhand in der Zeichnung dargestellter Ausführungsbeispiele näher erläutert. Es zeigen:
- Fig. 1: eine Draufsicht auf eine erfindungsgemäß aufgebaute Windel im aufgefalteten Zustand,
- Fig. 2: ein Schnitt entlang der Linie II-II in Fig. 1,
- Fig. 3 und 4: jeweils Schnitte entlang der Linien II-II jeweils weiterer Ausführungsformen,
- Fig. 5: eine Draufsicht auf ein weiteres Ausführungsbeispiel der Erfindung im aufgefalteten Zustand,
- Fig. 6: einen Schnitt entlang der Linie VI-VI in Fig. 5 und
- Fig. 7: eine Teildarstellung eines Schnitts eines nächsten Ausführungsbeispiels einer erfindungsgemäßen Windel.

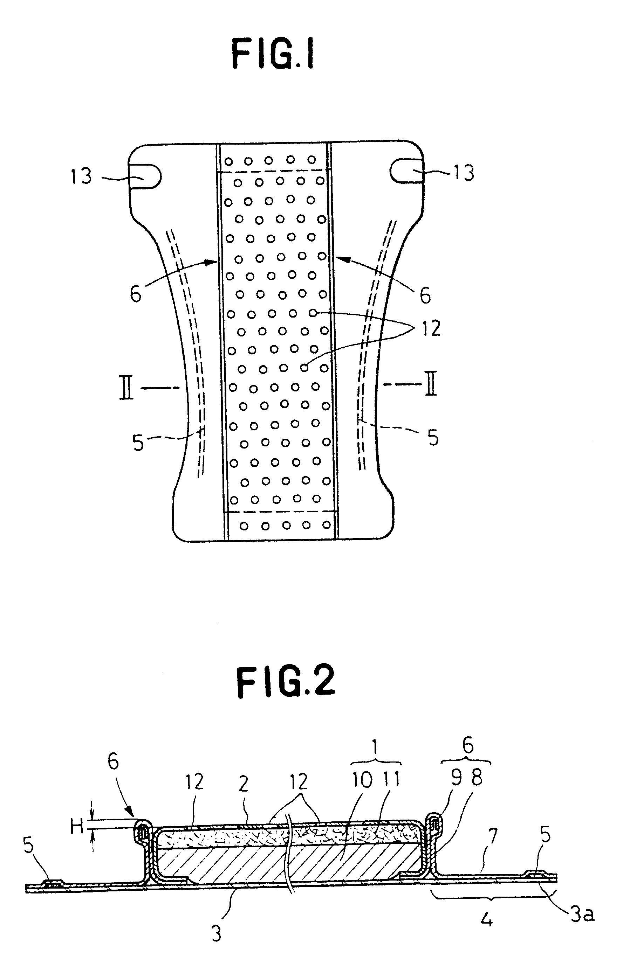

Wie in den Fig. 1 und 2 dargestellt, weist eine Windel einen Saugkern 1 einer vorbestimmten Dicke, eine wasserdurchlässige obere Lage 2, eine wasserundurchlässige rückseitige Lage 3, wasserundurchlässige Seitenklappen 4, elastische Bänder 5 und Leckschutzwände 6 auf.

Die obere Lage 2 bedeckt den Saugkern 1 an dessen Oberfläche und um die Randkanten herum. Die rückseitige Lage 3 bedeckt die Bodenfläche des Saugkerns 1 und erstreckt sich nach außen über die Randkante des Saugkerns 1 hinaus. Die Seitenklappen 4 umfassen einander gegenüberliegende Verlängerungen 3a der rückseitigen Lage 3 und wasserundurchlässige Lagen 7, die einerseits an den jeweiligen Oberflächen der seitlichen Verlängerungen befestigt sind und andererseits, in dem dargestellten Ausführungsbeispiel, die Seitenwände des Saugkerns bedecken.

Die elastischen Bänder 5 sind beispielsweise aus Natur- oder Synthetikgummi oder Kunststoff hergestellt und zwischen den zugeordneten Verlängerungen 3a und den Lagen 7, welche beiden Teile die jeweiligen Seitenklappen 4 bilden, eingeklebt. Um einen guten Sitz der Windel am Körper sicherzustellen, divergiert der Abstand zwischen einem Paar dieser elastischen Bänder 5 von einer vorderen Zone zu einer rückwärtigen Zone der Windel, wie es beispielsweise im aufgefalteten Zustand der Windel in Fig. 1 zu erkennen ist.

Die Leckschutzwände 6 bestehen aus der Lage 7, die nach oben vorsteht und dort die Form eines umgekehrten U oder Ω besitzt, in der ein in Längsrichtung elastisches Band 9 enthalten ist. Ein elastisches Band 9 ist an der Innenseite des auf diese Weise gebildeten Vorsprungs 8 eingeklebt, wobei die Leckschutzwand 6 als Gesamtheit entlang der sich gegenüberliegenden Seitenränder des Saugkerns über deren Oberfläche vorsteht. Vorzugsweise ist die Innenseite des Vorsprungs 8 intermittierend an dem Bereich der oberen Lage 2 befestigt, die sich entlang der einander gegenüberliegenden Seiten des Kerns 1 erstrecken, wobei die Befestigung zumindest in der Nähe der oberen Kante dieses Bereichs erfolgt.

Die Höhe H der Leckschutzwand, gemessen von der Oberfläche der oberen Lage 2, die der oberen Randkante der gegenüberliegenden Seiten des Kerns 1 entspricht, beträgt 3 - 20 mm, vorzugsweise 5 - 15 mm.

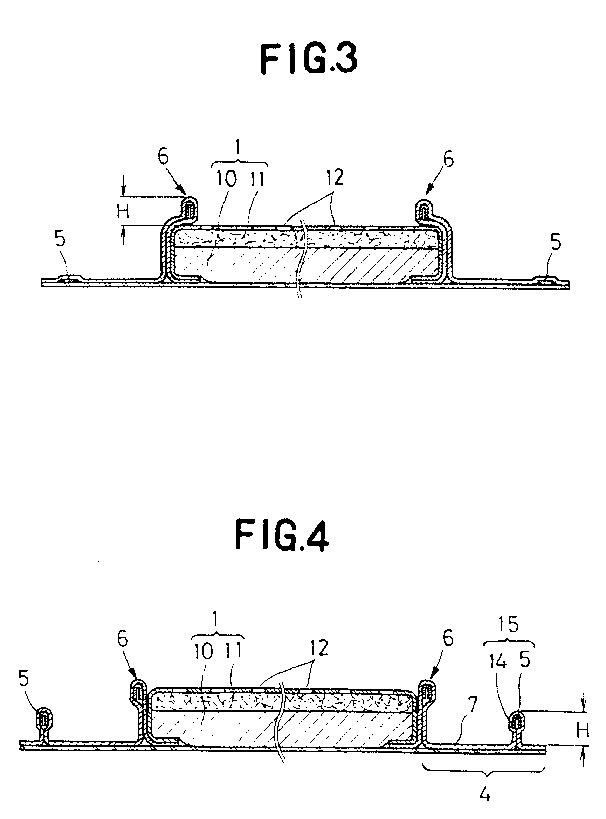

Die Leckschutzwände 6, dargestellt in Fig. 3, sind im Bereich der oberen Enden über die einander gegenüberliegenden Seiten des Kerns 1 nach innen versetzt. In diesem Fall sind die gefalteten Bereiche der jeweiligen Leckschutzwände 6 vorzugsweise auf der Oberfläche der oberen Lage 2 zumindest entlang den entsprechenden oberen Randkanten dieser Seiten befestigt.

Jedes elastische Band 9 kann aus einem Material ähnlich dem des Bands 5 bestehen. Ein Band oder Streifen eines solchen Materials wird in Querrichtung zu einer umgekehrten U-Form gedrückt und in der oberen Hälfte des Vorsprungs 8 eingelegt. Obwohl es nicht so wichtig ist, aus welchem besonderen Material die elastischen Bänder hergestellt sind und in welcher Form und ob jedes von ihnen in der oberen Hälfte des Vorsprungs 8 eingesetzt ist oder nicht, und ob jedes das Innere des Vorsprungs 8 vollausfüllt oder nicht, sind die elastischen Bänder 9 vorzugsweise in den jeweiligen Vorsprüngen 8 in dem oberen Bereich angeordnet, soweit die elastischen Bänder 9 nur die gewünschte Elastizität aufweisen.

Wesentlich ist, daß die Leckschutzwände in einer aufrechten Form an den oberen Rändern der sich gegenüberliegenden Seiten des Kerns 1 gehalten werden, unabhängig davon, aus welchem Material, welche Form und welchen Ort sie einnehmen, wobei das Aufrechterhalten unter der Zugkraft der elastischen Bänder 9 stattfindet, die die jeweiligen Leckschutzwände 6 bogenförmig aufrichten. Es sollte jedoch darauf hingewiesen werden, daß die in Längsrichtung einander gegenüberliegenden Enden der Leckschutzwände 6 nach außen umgeklappt und in einer solchen Lage befestigt sind, daß ein zwischen diesen beiden Enden definierter Abschnitt des elastischen Bands die Neigung erhält, nach außen zu kippen, wodurch eine unerwünschte Neigung des Bandes 9, nach innen zu kippen, wenn die Windel angelegt ist, verhindert wird.

Der Saugkern 1 weist eine absorbierende Rückhaltelage 10, bestehend aus einer flockigen Pulpe, faserigem Gewebe oder einem Faservlies mit darin eingemischten hochabsorbierenden Polymeren (nicht dargestellt) und einer Isolierlage 11 in Form einer Faserlage mit relativ niedriger Dichte großen Freiraum darin zu schaffen und um eine hohe kompressible federnde Rückbildungsrate unter Naßbedingung bereitzustellen. Die Isolierlage 11 ist zwischen der oberen Lage 2 und dieser absorbierenden Rückhaltelage 10 eingelegt und stellt eine Art Vorkammer dar, in die flüssige Rxkretion schnell ein- und hindurchdringen kann. Die Isolierlage 11 dient nicht zu dazu, die Möglichkeit zu vermindern, daß eine Menge flüssiger Exkretion, die bereits von der absorbierenden Rückhaltelage 10 aufgesaugt worden ist, wieder zurück zur Oberfläche dieser Rückhaltelage 10 zu fließen und durch die Oberlage 2 zu sickern, sondern bildet einen Auffangraum und verleiht dem Saugkern 1 auch eine kissenartige Natur, um den Träger frei von jeglichem Unwohlgefül zu halten.

Um derartige Funktionen adequat auszuüben, weist die Isolierlage 11 vorzugsweise Fasern mit einer Länge zwischen 35 - 100 mm auf, die an gegenseitigen Kreuzungspunkten miteinander verschweißt sind, um die Gestalt eines Gewebes zu erhalten. Einzelne Fasern sind vorzugsweise hydrophobische Fasern, von denen zumindest 50 Gew.-% wasserabsorbierend ("schweißabsorbierend"), insbesondere schweißabsorbierende Polyesterfasern sind. Um das Faserverschweißen durchzuführen, enthalten die Fasern vorzugsweise annähernd 30 Gew.-% niedrigschmelzender (110 - 200°C) Polyesterfasern, Polyethylenfasern, Polypropylenfasern oder konjugative Fasern (Seite an Seite, Kernlagertyp) aus Polyethylen/Polypropylen-Polyester mit niedrigem Schmelzpunkt/normales Polyester. Die Isolierlage besitzt eine Dicke von 2 - 10 mm, gemessen nachdem es unter einer Last von 3 g/cm² für 1 Minute gehalten wurde, einem Gewicht pro Flächeneinheit von 20 - 80 g/m², eine federnde Kompressionsrückbildungsrate unter Feuchtigkeitsbedingungen von mehr als 30%, vorzugsweise höher als 50% und ein Feinheitsgrad von 3 - 13 d.

Die obere Lage 2 wird von ineinander verschlungenen Fasern in Gestalt eines Faservlieses gebildet und ist mit einer Vielzahl von regelmäßig angeordneten Öffnungen 12 versehen, die über die gesamte Oberfläche verteilt sind. Die Öffnungen 12 werden durch Aufteilen von Fasern gebildet. Jede dieser Öffnungen 12 besitzt vorzugsweise einen definierten Umfang, ohne daß Fasern sich über diese Öffnung hinwegerstrecken.

Eine solche obere Lage 2 wird beispielsweise durch ein Verfahren erhalten, bei welchem eine faserige Lage auf ein Trägerteil mit einer Vielzahl darauf vorgesehener Vorsprünge aufgelegt, wobei ein Hochgeschwindigkeitswasserstrahl von oben auf die faserige Lage gespritzt wird, um die gewünschte Faserverschlingung zu erzielen und um auch zu bewirken, daß die Fasern sich an den jeweiligen Vorsprüngen zur Seite bewegen. Die Öffnungen 12 sind im vorliegenden Fall kreisförmig und weisen eine Öffnungsfläche zwischen 7 - 50 mm², einen Durchmesser zwischen 2 - 10 mm, einen Reihenabstand von 6 - 20 mm und ein Gesamtöffnungsverhältnis bezüglich der Gesamtoberfläche von 15 - 70% auf (vorzugsweise).

Die obere Lage 2 ist so aufgebaut, daß Körperflüssigkeiten, wie Urin und Schweiß, frei durch sie hindurchtreten, ohne die Oberfläche der nicht mit Öffnungen versehenen Bereich zu tränken, der zuvor mit einem wasserabstoßenden Mittel bekannter Art behandelt wurde. Es wird bevorzugt, daß 70 oder mehr Gew.-% Rayonfasern mit einem Feinheitsgrad von 0,5 - 3 d in der oberen Lage 2 enthalten sind. Jedoch können 70 oder mehr Gew.-% der wasserabsorbierenden Fasern oder gewöhnliche hydrophobische Fasern enthalten sein, und eine der am meisten bevorzugten Fasern ist die Polyesterfaser mit einem Feinheitsgrad von 1 - 3 d. Die obere Lage 2 weist ein Gewicht pro Flächeneinheit von ca. 15 - 45 g/m² auf.

Die rückseitige Lage 3 weist einen luftdurchlässigen, flüssigkeitsundurchlässigen Kunststoffilm oder ein Laminat aus letzterem und einem faserigen Vlies auf. Die wasserundurchlässige Lage 7 bildet einen Teil jeder Seitenklappe 4 und besteht vorzugsweise aus einer luftdurchlässigen Lage eines Faservlies, insbesondere spinngebundenes Faservlies aus Polypropylen, das wasserabstoßend behandelt worden ist.

Die auf diese Weise aufgebaute Windel wird wie im Fall von bekannten Windeln zusammengebaut und mittels Befestigens freier Enden eines Bandbefestigers 13, die an gegenüberliegenden Seiten des rückwärtigen Bereichs auf Höhe der Hüftlinie vorgesehen sind, an gegenüberliegenden Seiten der rückseitigen Lage 3 im vorderen Bereich angelegt.

In Fig. 4 ist ein Ausführungsbeispiel der Erfindung dargestellt, wobei die Lage 7, die ein Teil jeder Seitenklappe 4 bildet, teilweise in umgekehrter U- oder Ω-Form vorsteht, wobei ein auf diese Weise gebildeter Vorsprung 17 im Inneren ein elastisches Band 5 aufweist, das im Querschnitt zu einem umgekehrten U gekrümmt ist, um eine Leckschutzwand 15 zu bilden. Diese Leckschutzwand 15 ist ebenfalls, wie im Falle der oben erwähnten Leckschutzwand 6 aufrechtstehend und weist eine Höhe H auf, wobei die sich in Längsrichtung einander gegenüberliegenden Enden vorzugsweise nach außen umgefaltet und befestigt sind.

In den Fig. 5 und 6 ist ein weiteres Ausführungsbeispiel der Erfindung dargestellt, wobei jede der Seitenklappen 4 von der rückwärtigen Zone zur Vorderzone abgeschrägt ist und jedes der elastischen Bänder 5 zwischen einer Verlängerung 3a der Rückseite, die die Seitenklappe 4 bildet und der Lage 7 eingeschlossen. Der Abstand zwischen einem Paar elastischer Bänder 5 ist entsprechend von dem rückwärtigen Bereich zum vorderen Bereich der Windel verjüngt, wobei die vorderen Abschnitte des Bands 5 auf den Bodenrändern der einander gegenüberliegenden Seiten des Saugkerns 1 liegen und diese schneiden. Diese Frontabschnitte können jedoch, anstatt diese Seiten zu schneiden, neben diesen zu liegen kommen.

Es versteht sich, daß die einander gegenüberliegenden Seitenränder der oberen Lage 2, wie in Fig. 7 zu erkennen ist, sich nach außen erstrecken können. Des weiteren können die Leckschutzwände 6 und 15 lediglich im Schrittbereich der Windel angeordnet sein.

## Patentansprüche

1. Wegwerfwindel mit einem Saugkern (1) vorbestimmter Dicke, einer zumindest die obere Fläche des Saugkerns (1) bedekkende wasserdurchlässige obere Lage (2), einer zumindest die Boden- oder rückwärtige Fläche des Saugkerns bedeckende wasserundurchlässige rückseitige Lage (3), mit wasserundurchlässigen Seitenklappen (4), die sich an gegenüberliegenden Seiten des Saugkerns (1) erstrecken, ersten elastischen Mitteln (5), die entlang der jeweiligen Seitenklappen elastische Einkräuselungen bilden und mit nach innen (zum Körper) ragenden umgekehrten U-oder Ω-förmig ausgebildeten Schutzwänden (6) dadurch **gekennzeichnet,** daß
- die Schutzwände als wasserdichte Leckschutzwände (6) ausgebildet sind,
- die Leckschutzwände (6) den Saugkern (1) an dessen Längsseiten bedecken und mit einem Teil ihrer Höhe den Saugkern nach oben überragen
- der den Saugkern nach oben überragende Teil U- oder Ω-förmig ausgebildet ist und
- in diesem Teil ein in umgekehrter U- oder Ω-Form gekrümmtes zweites elastisches Band (9) angeordnet ist.

2. Windel nach Anspruch 1, dadurch **gekennzeichnet,** daß die Leckschutzwände (6) jeweils eine Höhe von zumindest 3 mm, gemessen von der Oberfläche der oberen Lage (2) am oberen Rand der sich gegenüberliegenden Seiten des Saugkerns (1) beträgt.

3. Windel nach Anspruch 1 oder 2, dadurch **gekennzeichnet,** daß die Leckschutzwand (6) intermittierend an der oberen Lage (2) befestigt ist, die sich über die gegenüberliegenden Seiten des Saugerkerns (2) erstreckt.

4. Windel nach mindestens einem der Ansprüche 1 bis 3, dadurch **gekennzeichnet,** daß die Leckschutzwände von einem Abschnitt einer wasserundurchlässigen Lage (7) gebildet werden, die Bestandteil einer seitenklappe ist.

5. Windel nach mindestens einem der Ansprüche 1 bis 4, **dadurch gekennzeichnet,** daß der Abstand zwischen einem Paar erster elastischer Bänder (5) sich von einem rückwärtigen Bereich zu einem vorderen Bereich der Winkel verringert.

6. Windel nach mindestens einem der Ansprüche 1 bis 5, **dadurch gekennzeichnet,** daß die ersten elastischen Bänder (5) so angeordnet sind, daß ein Abstand zwischen ihnen von dem rückwärtigen Bereich zum vorderen Bereich der Windel graduell vermindert wird und die vorderen Abschnitte neben den vorderen Abschnitten des zweiten elastischen Bands zu liegen kommen, oder diese schneiden.

7. Windel nach mindestens einem der Ansprüche 1 bis 6, **dadurch gekennzeichnet,** daß eine obere Lage des Saugkerns (1) eine Isolierlage (11) mit einer Faserdichte aufweist, die geringer ist als die untere Lage, und daß darin ein großer Freiraum enthalten ist.

8. Windel nach mindestens einem der Ansprüche 1 bis 7, **dadurch gekennzeichnet,** daß die obere Lage (2) zumindest in dem Bereich des Saugkerns (1) mit einer Vielzahl von Öffnungen versehen ist.

## Claims

1. Disposable nappy with an absorbent body (1) of predetermined thickness, a water-permeable upper layer (2) covering at least the upper surface of the absorbent body (1), a water-impermeable rear layer (3) covering at least the base or rear surface of the absorbent body, with water-impermeable side flaps (4) which extend on opposite sides of the absorbent body (1), first elastic means (5) which form elastic crimps along the respective side flaps, and with inwardly- (towards the body) extending inverted U or Ω-shaped protective walls (6), characterised in that
- the protective walls are formed as watertight leak-preventing walls (6),
- the leak-preventing walls (6) cover the absorbent body (1) at its long sides and extend upwards beyond the absorbent body with a part of their height,
- the part extending upwards beyond the absorbent body is U-or Ω-shaped, and
- in this part there is arranged a second elastic band (9) twisted in an inverted U or Ω-shape.

2. Nappy according to Claim 1, characterised in that the leak preventing walls (6) each have a height of at least 3mm, measured from the surface of the upper layer (2) at the upper edge of the opposite sides of the absorbent body (1).

3. Nappy according to Claim 1 or 2, characterised in that the leak-preventing wall (6) is intermittently secured to the upper layer (2), which extends over the opposite sides of the absorbent body (1).

4. Nappy according to at least one of Claims 1 to 3, characterised in that the leak-preventing walls are formed from a portion of a water-impermeable layer (7) which is part of a side flap.

5. Nappy according to at least one of Claims 1 to 4, characterised in that the separation between a pair of first elastic bands (5) reduces from a rear region to a fore region of the nappy.

6. Nappy according to at least one of Claims 1 to 5, characterised in that the first elastic bands (5) are so arranged that a separation between them gradually reduces from the rear region to the fore region of the nappy, and the fore parts come to lie near the fore parts of the second elastic bands, or cross them.

7. Happy according to at least one of Claims 1 to 6, characterised in that an upper layer of the absorbent body (1) is provided with an isolating layer (11) with a fibre density which is lower than the underlayer, and in that a large free space is contained therein.

8. Nappy according to at least one of Claims 1 to 7, characterised in that the upper layer (2) is provided at least in the region of the absorbent body (1) with a plurality of openings.

## Revendications

1. Couche jetable comportant un noyau absorbant (1) d'épaisseur déterminée, un pli supérieur perméable (2) recouvrant au minimum la surface du noyau absorbant (1), un pli arrière imperméable (3) recouvrant au minimum la face arrière ou le fond du noyau absorbant, comportant des pattes latérales imperméables (4), qui s'étendent contre les côtés opposés du noyau absorbant (1), une première bande élastique (5), qui, le long de chaque patte latérale constitue des frisures élastiques et comportant des parois de protection (6), en forme de U renversé ou de Ω , dépassant vers l'intérieur (vers le corps), caractérisée en ce que :
- les parois de protection sont des parois imperméables anti-fuites (6),
- les parois anti-fuites (6) recouvrent le noyau absorbant (1) sur ses côtés longitudinaux et le dépassent vers le haut avec une partie de leur hauteur,
- la partie, qui dépasse vers le haut le noyau absorbant, a la forme d'un U ou d'un Ω , et
- dans cette partie, est placée une deuxième bande élastique (9) recourbée en forme de U renversé ou de Ω .

2. Couche selon la revendication 1, caractérisée en ce que la hauteur respective des parois anti-fuites (6), mesurée à partir de la surface du pli supérieur (2) au bord supérieur des côtés opposés du noyau absorbant (1), s'élève au minimum à 3 mm.

3. Couche selon la revendication 2 ou 3, caractérisée en ce que la paroi anti-fuites (6) est fixée par intermittence au pli supérieur (2), qui s'étend sur les côtés opposés du noyau absorbant (1).

4. Couche selon l'une au moins des revendications 1 à 3, caractérisée en ce que les parois anti-fuites sont formées par un morceau d'un pli imperméable (7), lequel est un constituant d'une patte latérale.

5. Couche selon l'une au moins des revendication 1 à 4, caractérisée en ce que la distance entre deux des premières bandes élastiques (5) diminue en allant de la zone arrière à la zone avant de la couche.

6. Couche selon l'une au moins des revendications 1 à 5, caractérisée en ce que les premières bandes élastiques (5) sont placées de manière que leur intervalle mutuel diminue graduellement de la zone arrière à la zone avant de la couche et que leurs tronçons avant arrivent à côté des tronçons avant de la deuxième bande élastique ou la recoupent.

7. Couche selon l'une au moins des revendications 1 à 6, caractérisée en ce que la strate supérieure du noyau absorbant (1) est un matelas isolant (11), comportant une densité de libres, qui est plus faible que celle de la strate inférieure et en ce que, là, un plus grand espace libre est conservé.

8. Couche selon l'une au moins des revendications 1 à 7, caractérisée en ce que le pli supérieur (2) est, au moins dans la zone du noyau absorbant (1), pourvu d'une pluralité d'ouvertures.
